# EUROPEAN PATENT APPLICATION

(11) **EP 4 356 921 A1**
(43) Date of publication of application: **24.04.2024**
(21) Application number: 22921741.9
(22) Date of filing: 27.12.2022
(51) Int. Cl.: A61K 38/30, A61P 25/22, A61P 25/24, A61P 25/28

(54) **USE OF INSULIN-LIKE GROWTH FACTOR BINDING PROTEIN 2 IN PREPARATION OF DRUG FOR TREATING NEURODEMYELINATING DISEASES**

(30) Priority: 21.01.2022 CN 202210074291
(71) Applicant: Shanghai Mental Health Center (Shanghai Psychological Counselling Training Center), Shanghai 200030 (CN)
(72) Inventor: WANG, Zhen, Shanghai 200030 (CN); SHI, Dongdong, Shanghai 200030 (CN); ZHANG, Yingdan, Shanghai 200030 (CN)
(74) Representative: Dummett Copp LLP
(86) International application number: PCT/CN2022/142411
(87) International publication number: WO 2023/138320

(57) **Abstract**

The present invention relates to a use of insulin-like growth factor binding protein 2 in a preparation of drugs for treating neurological demyelinating diseases. IGFBP2 intervention can significantly increase myelination levels and improve psychiatric symptoms caused by stress demyelination.

## Description

### Cross referencing of related applications

This application claims the priority of the Chinese invention patent application with application number 202210074291.3 filed on January 21, 2022, and its content is incorporated into this application by reference.

### Field of the invention

The present invention relates to the field of drug preparation, in particular to use of insulin-like growth factor binding protein 2 in the preparation of drugs for treating neurological demyelinating diseases.

### Background of the invention

Myelin sheath is a layer of membrane that wraps around the axons of nerve cells, consisting of Schwann cells and myelin sheath cell membranes. Its function is insulation, preventing nerve electrical impulses from being transmitted from the neuron axon to another neuron axon. Demyelination refers to the damage to the myelin sheath that occurs after its formation. Demyelinating diseases are a type of acquired diseases with different etiologies and clinical manifestations, but with similar characteristics. The pathological changes characterized by the loss of myelin sheath in nerve fibers while nerve cells remain relatively intact. Due to the fact that the normal transmission of nerve impulses depends on the insulation properties of the myelin sheath around the axon, myelin dysfunction or changes in the myelin sheath can impair learning and adaptive behavior. Research data shows that even small changes in the myelin sheath throughout the entire lifecycle can have an impact on the circuits and systemic mechanisms involved in cognition and behaviour. The function of myelin may be more extensive than previously assumed: dysfunctional or maladaptive myelin formation may lead to neurodegenerative diseases and neuropsychiatric disorders, which are now considered to have a neuronal basis.

Many human and animal research reports suggest that demyelination of white matter in specific brain regions of the central nervous system may be an important pathophysiological feature of mental disorders. White matter serves as an important neural matrix connecting the distributed brain system. Neuroimaging studies have revealed the abnormal microstructure of human white matter exposed to stress. According to reports, changes in white matter demyelination are associated with poor stress adaptation and recovery ability. Meanwhile, strong evidence from anxiety patients suggests a negative correlation between demyelination of white matter in prefrontal cortex related pathways and trait anxiety scores. Meanwhile, loss of myelin sheath in mPFC was also observed in various animal stress models with anxiety like and depression like behaviors. We speculate that when myelin damage is repaired in a timely and effective manner, the risk of developing mental disorders such as anxiety, depression, and cognitive impairment will be reduced.

Insulin-like growth factor binding protein 2 (IGFBP2), as one of the six major members of IGF binding proteins, is a growth factor with both autocrine and paracrine properties. Research has shown that IGFBP2 not only participates in brain growth and development, but also regulates neuronal plasticity, spatial learning and memory, and advanced cognitive functions such as information processing. In addition, IGFBP2 is widely present in the central nervous system and highly expressed in the developing brain, secreted in primary astrocytes and neurons. Its concentration in the periphery is relatively low, and the molecular weight of IGFBP2 is 36 kD, which can cross the blood-brain barrier (BBB). However, there is currently no relevant research to prove that IGFBP2 can treat mental disorders after demyelination.

### Summary of the invention

The purpose of the present invention is to overcome the shortcomings of the prior art and provide a use of insulin-like growth factor binding protein 2 in the preparation of drugs for the treatment of neurological demyelinating diseases.

The present invention provides a use of insulin-like growth factor binding protein 2 in the preparation of drugs for the treatment of neurological demyelinating diseases.

Preferably, the use of insulin-like growth factor binding protein 2 in the preparation of drugs that enhance myelination levels is provided. Wherein, improving the level of myelination includes aspects such as myelination status and myelination quantity.

Alternatively, the use of insulin-like growth factor binding protein 2 in the preparation of drugs for the treatment of demyelinating diseases.

Preferably, said neurological demyelinating disease (demyelinating disease) comprises symptoms of anxiety, depression, and cognitive impairment.

The present invention also provides an evaluation method for the use of insulin-like growth factor binding protein 2 in the preparation of drugs for treating neurological demyelinating diseases, comprising the following steps:
Step 1: Establish a demyelinating model;
Step 2: Select evaluation criteria;
Step 3: Evaluate the intervention effect of IGFBP2.

### Brief Description Of The Drawings

Figure 1a shows the process of drug demyelination modelling and IGFBP2 intervention.
Figure 1b shows the process of stress demyelination modelling and IGFBP2 intervention.
Figures 2a and 2b demonstrate the effect of IGFBP2 intervention on animal myelination levels through drug demyelination validation.
Figures 3a-3e demonstrate the effect of IGFBP2 intervention on animal myelination levels through stress demyelination verification.
Figures 4a-4f show the validation of the effect of IGFBP2 intervention on animal behavior after stress demyelination through drug demyelination.
Figures 5a-5h show the validation of the effect of IGFBP2 intervention on animal behavior after stress demyelination through stress demyelination.

### Detailed description of the invention

In order to better describe the technical content of the present invention and verify the role of IGFBP2 in the treatment of neurological demyelinating diseases, further description will be given in conjunction with specific embodiments below.

### Establishing a demyelinating rat model

SD rats are raised in a 12-hour light/dark rhythmic environment and can freely access water and food. All behavioral experiments are conducted in the light rhythm of animals.

### Drug demyelination:

Firstly, SD rats were used as models and isoflurane (R510-22, RWD China, 4-5% induction, 1-2% maintenance) was used to induce anesthesia. After deep anesthesia, 1 µ L of 1% lysolecithin (LPC, Sigma Aldrich) was surgically dissolved in saline using a 33 gauge syringe needle (Hamilton) at the following three-dimensional coordinates, and the control group used the same volume of saline. The liquid is injected into both sides of the mPFC:+2.7 millimeters; The middle side of the anterior fontanelle, 0.4 millimeters; Below the surface of the dorsal and ventral dura mater, 3.3 millimeters. The volume of lysolecithin is 1 µ L, and the unilateral injection time is 15 minutes. After injection, let the needle stand for 5 minutes to fully absorb the medication. Slowly adjust the Z-axis knob to pull the needle upwards. Suture the skin to complete brain targeted injection.

### Stress Demyelination:

The stress mode is: long term unpredictable chronic stress (CUMS), which involves using different stress modes and conducting chronic stress for 5 weeks, once a day. The specific stress modes are shown in Table 1.

**Table 1. CUMS stress patterns**

| Stress mode | description | Duration |
|---|---|---|
| Cage tilted | The cage is tilted 45 ° | 12 H |
| Strange Mouse | Place an unfamiliar animal in a cage | 12 H |
| Food or water deprivation | Take away food or water | 12 H |
| Wet cage | 100 grams of padding and 200 milliliters of water | 12 H |
| Continuous lighting | Lighting at night | 12 H |
| restraint | Placed in a tube to restraint | 2 H |
| Pinch the tail | Use a clip at the tip of the tail 1 centimeter away | 1 min |
| Ice water swimming | Placed in ice water at 6 °C | 5 min |

### Intervention of IGFBP2

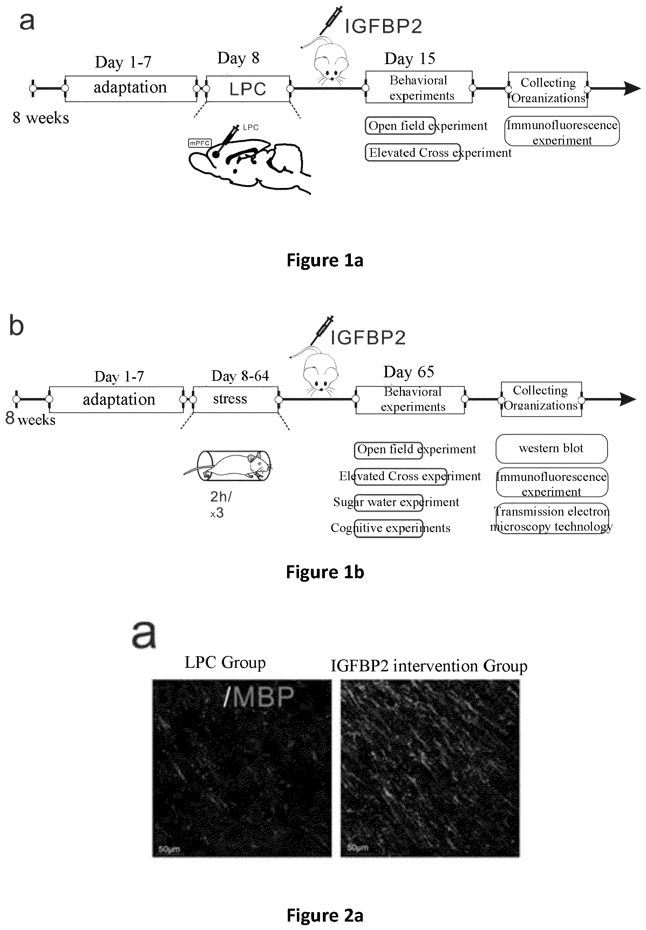

IGFBP2 Intervention for drug induced demyelination: After 4 days of LPC injection surgery, demyelination is completed and IGFBP2 intervention is performed. Conduct behavioral testing 8 days after surgery. The experimental process is shown in Figure 1a.

IGFBP2 Intervention for stress induced demyelination: One day after the end of stress, IGFBP2 intervention is performed. Conduct behavioral testing on the second day. The experimental process is shown in Figure 1b.

### Evaluate the effectiveness of IGFBP2 intervention

Firstly, observe the morphology of the myelin sheath through immunohistochemistry and electron microscopy experiments to determine whether IGCBP2 reverses drug or stress-induced demyelination.

Immunohistochemical experiment: Rats were fixed on a separate anatomical plate, first perfused with 1xPBS, then perfused with 4% paraformaldehyde (PFA), and fixed overnight in 4% PFA at 4 °C. Then dehydrate the brain tissue with 30% sucrose until it completely sinks into the bottom of a 50 mL tube. After OCT embedding, store the brain in a -80 °C freezer. Cut 30 µm coronal sections using a frozen slicer. Wash the mPFC region slices with PBS three times, each time for 5 minutes. Incubate at room temperature in PBS containing 4% goat serum and 0.1% Triton X-100 for 1 hour, followed by incubation with first anti MBP antibody (1:500) at 4 °C overnight. Wash 5 times at room temperature in PBS and 0.1% Triton X-100 (PBST) for 5 minutes. Incubate fluorescent secondary antibody with Alexa Fluor 488 goat anti rabbit diluted 1:1000 at room temperature. Then wash 5 times in the dark room in PBST for 5 minutes each time. Slices were re stained with DAPI and then sealed. All images were obtained using confocal microscopy (OLYMPUS) and analyzed using Fuji (NIMH, Bethesda MD, USA).

Electron microscopy experiment: used to measure the thickness of myelin sheath and the thickness and length of postsynaptic dense body (PSD) in the mPFC region. Trim the mPFC brain region to include 1 mm³ of the area. Cut the slices at 60 nm using a Leica EM UC7 ultra-thin slicer and stain with uranyl acetate and lead citrate. Collect images of each rat using a transmission electron microscope (Tecnai G2 SpiritBiowin). The G ratio is determined by dividing the diameter of the axon by the diameter of the entire myelinated fiber. Fuji (NIMH, Bethesda MD, USA) is used to measure axon diameter, myelin sheath fiber diameter, and PSD thickness and length.

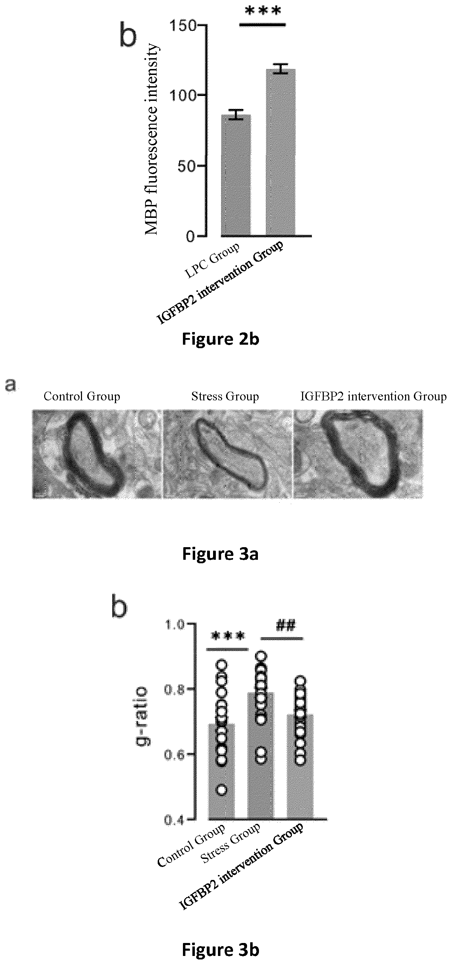

As shown in Figures 2a and 2b, the intervention effect of IGFBP2 was observed through immunohistochemistry after demyelination. Figures 2a and 2b show the level of myelination in the mPFC brain region. After drug demyelination, the expression of MBP significantly decreased and the level of myelination significantly decreased. After IGFBP2 intervention, the level of myelination significantly increased. Two factor analysis of variance, *** represents P<0.001, ** represents P<0.001, and * represents P<0.05.

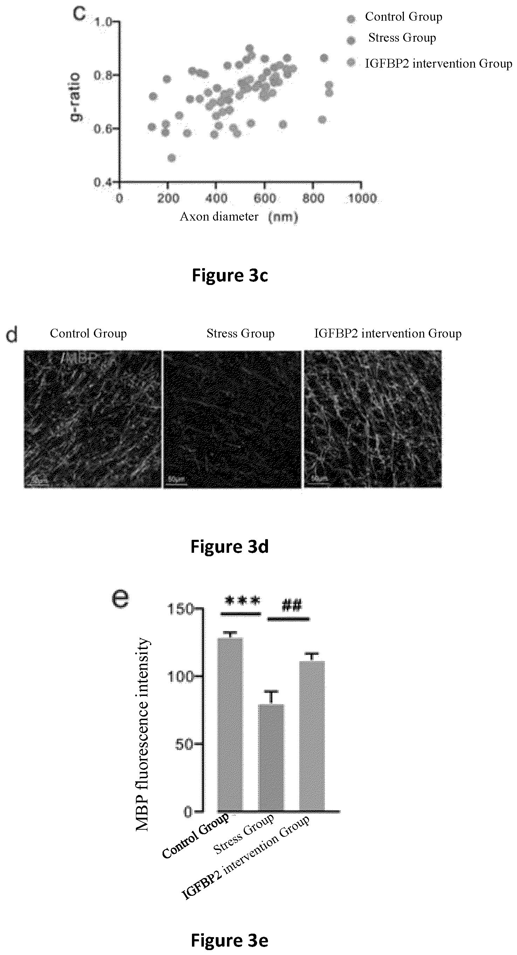

As shown in Figures 3a-3e, after stress induced demyelination, electron microscopy experiments showed that stress induced demyelination, and IGFBP2 intervention improved myelination; The results of immunofluorescence experiments showed that stress significantly reduced the fluorescence intensity of MBP, while the intervention of IGFBP2 restored the fluorescence intensity. Wherein, *** represents P<0.001, ** represents P<0.001, * represents P<0.05, * represents control group vs stress group; # represents control group vs IGFBP2 intervention group.

Based on the experimental data, it is supported that IGFBP2 intervention can significantly increase myelination and improve the level of myelination in demyelinating diseases.

Further observe the improvement of psychiatric symptoms in demyelinating diseases through behavioral experiments with IGFBP2 intervention.

Verify the intervention effect of IGFBP2 on psychiatric symptoms after demyelination through behavioral testing. All behavioral experiments were conducted during rat light exposure time. Before conducting behavioral experiments, the rats were placed in a behavioral room and acclimated for 2 hours. During each experiment, after completing each mouse experiment, wipe with 70% alcohol to avoid the impact of odor on the next mouse. Handle rats for a few minutes every day before behavioral school, and adapt for at least 4 days. The behavioral experiment used sugar water preference experiment, tail suspension experiment, forced swimming experiment to evaluate depressive like behavior, open field experiment and elevated cross maze experiment to evaluate anxiety like behavior, as well as new object recognition experiment and Barnes maze experiment to evaluate cognitive behavior.

Sugar preference experiment: Two days in advance, double bottle adaptation, one bottle of drinking water and one bottle of 1% sucrose. On the third day of testing, the animals were dehydrated for 6 hours before testing, and their preference for sugar water (%) was (sugar water consumption/(sugar water + regular water consumption)) * 100.

Open field experiment: Place the experimental animals in a box of 50 cm × 50 cm, with a camera time of 10 minutes, and calculate their time in the central area.

Elevated cross maze experiment: Place rats in the cross maze from the central grid facing the open arm direction, and record the number of times they enter the open arm, the duration of their stay, the number of times they enter the closed arm, and the duration of their stay for 5 minutes.

Water maze experiment: Rats are trained for four days, recording the time when they find the platform and the last day they spend in the target quadrant.

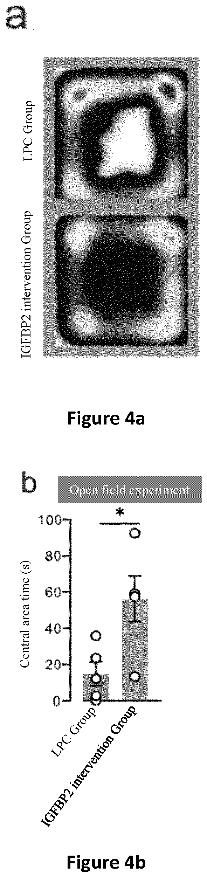

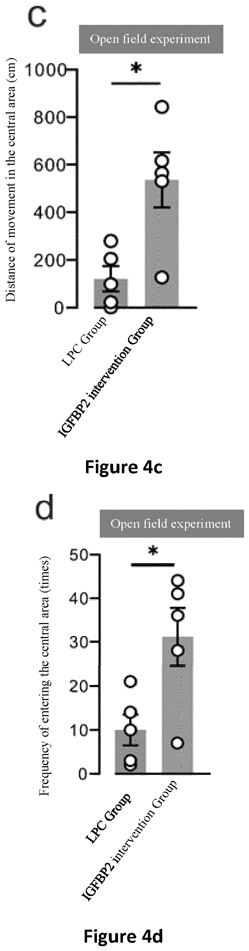

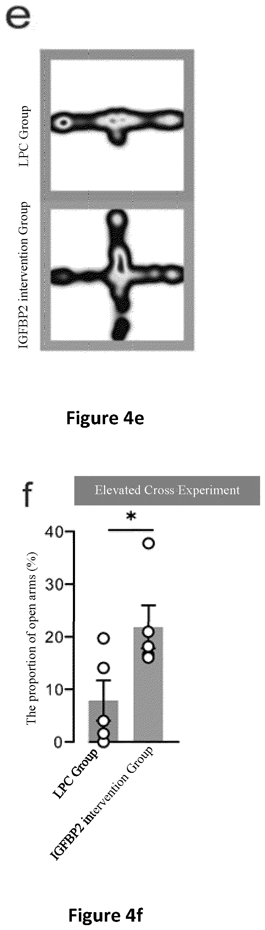

For drug demyelinating animals, Figures 4a-4d show the results of the open field experiment, while Figures 4e and 4f show the results of the elevated cross maze experiment. The proportion of time for animals in the open arm is: open arm time/(open arm time + closed arm time). Using a two factor analysis of variance, *** represents P<0.001, ** represents P<0.001, and * represents P<0.05.

The results of the open field experiment showed that the animals in the demyelination group (LPC group) spent significantly less time in the central area than the control group, while after IGFBP2 intervention, the animals in the demyelination group (IGFBP2 intervention group) spent significantly more time in the central area.

The results of the elevated cross maze experiment showed that the animals in the demyelinating group (LPC group) had significantly lower open arm time than the control group. The intervention of IGFBP2 significantly increased the open arm time of demyelinating group (IGFBP2 intervention group).

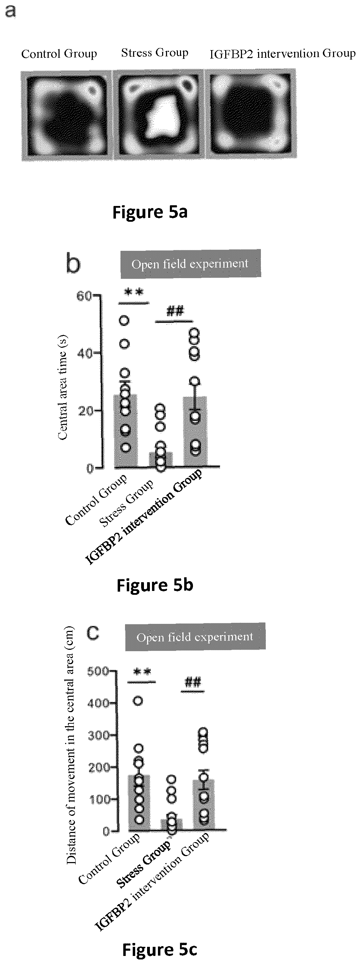

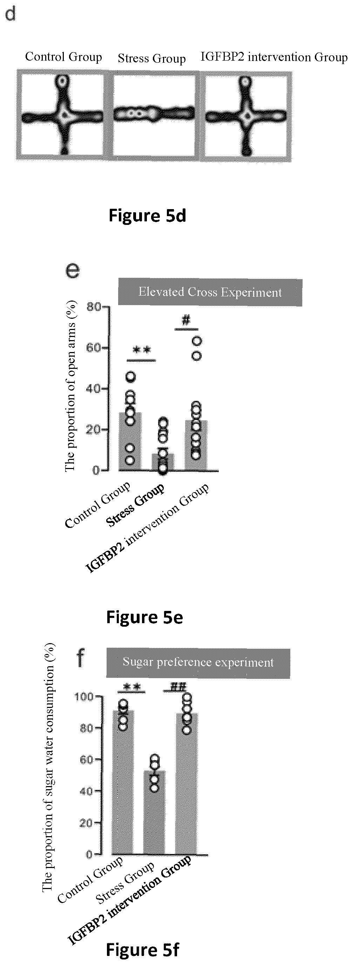

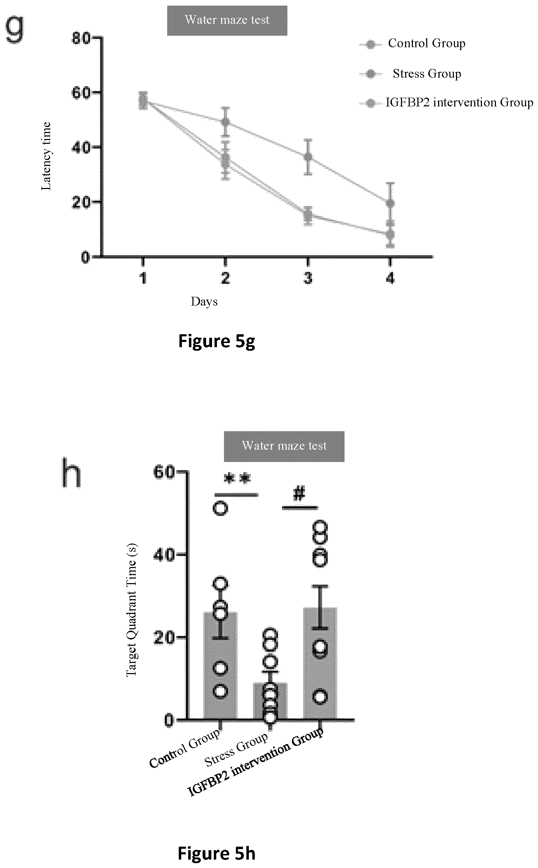

For stress demyelinating animals, stress leads to demyelination of the mPFC brain area. Figures 5a-5c show the results of the open field experiment, while Figures 5d and 5e show the results of the elevated cross maze experiment. The proportion of open arm time in animals is: open arm time/(open arm time + closed arm time), and Figure 5f shows the results of the sugar water preference experiment. The proportion of sugar water consumption in animals is: sugar water consumption/(sugar water consumption + water consumption), Figures 5g and 5h show the results of the water maze experiment, showing the time it takes for animals to find the platform during the training process. Using a two factor analysis of variance, *** represents P<0.001, ** represents P<0.001, and * represents P<0.05.

The results of the open field experiment showed that in the control animals, the intervention of IGFBP2 did not affect its time in the central area. The time of animals in the stress group in the central area was significantly lower than that in the control group, while after IGFBP2 intervention, the time of animals in the stress group in the central area was significantly increased (P<0.0001).

The results of the elevated cross maze experiment showed that the stress group animals had significantly lower open arm time than the control group. The intervention of IGFBP2 did not affect the duration of arm opening in the control group animals. However, the intervention of IGFBP2 significantly increased the time for stress group animals to open their arms.

The results of the sugar preference experiment showed that the sugar preference value of the stress group animals was significantly lower than that of the control group animals, showing a significant loss of pleasure behavior. The intervention of IGFBP2 did not affect the sugar preference value of the control group animals, but significantly increased the sugar preference value of the stress group animals.

The results of the water maze experiment showed that animals in the stress group had to spend more time finding the platform during the training process, but after IGFBP2 intervention, the time for animals in the stress group to find the platform significantly decreased.

The results of open field experiments and elevated cross maze experiments showed that IGFBP2 can effectively improve anxiety like behavior caused by demyelination. The results of the sugar preference experiment showed that IGFBP2 intervention can effectively improve depressive like behavior caused by demyelination. The results of the water maze experiment indicate that IGFBP2 intervention can effectively improve cognitive impairment caused by demyelination.

In summary, it supports that IGFBP2 intervention can significantly increase myelination levels and improve psychiatric symptoms caused by stress demyelination.

In this specification, the present invention has been described with reference to its specific embodiments. However, it is evident that various modifications and transformations can still be made without departing from the spirit and scope of the present invention. Therefore, the specification is considered explanatory rather than restrictive.

## Claims

1. Use of insulin-like growth factor binding protein 2 in a preparation of drugs for treatment of neurological demyelinating diseases.

2. The use according to claim 1, **characterized in that** insulin-like growth factor binding protein 2 is used in the preparation of drugs that enhance myelination levels.
